(19) Europäisches Patentamt
European Patent Office
Office européen des brevets

(11) **EP 2 604 209 B1**

(12) **EUROPÄISCHE PATENTSCHRIFT**

(45) Veröffentlichungstag und Bekanntmachung des Hinweises auf die Patenterteilung:
**02.08.2017 Patentblatt 2017/31**

(51) Int Cl.:
*A61B 18/14* ^(2006.01)     *A61B 18/00* ^(2006.01)
*A61B 90/00* ^(2016.01)

(21) Anmeldenummer: **12192916.0**

(22) Anmeldetag: **16.11.2012**

(54) **Spülkathetereinrichtung und Ablationsanordnung**

Irrigation catheter device and ablation system

Cathéter d'irrigation et système d'ablation

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.12.2011 US 201161569803 P**

(43) Veröffentlichungstag der Anmeldung:
**19.06.2013 Patentblatt 2013/25**

(73) Patentinhaber: **VascoMed GmbH**
**79589 Binzen (DE)**

(72) Erfinder:
• **Boomsma, Kevin**
**8800 Talwill (CH)**
• **Kiefer, Andreas**
**79211 Denzlingen (DE)**
• **Kaufmann, Ralf**
**79540 Lörrach (DE)**
• **Knorr, Stefan**
**10119 Berlin (DE)**
• **Weiss, Ingo**
**12435 Berlin (DE)**

(74) Vertreter: **Galander, Marcus et al**
**Biotronik SE & Co. KG**
**Corporate Intellectual Properties**
**Woermannkehre 1**
**12359 Berlin (DE)**

(56) Entgegenhaltungen:
**EP-A1- 2 343 022      EP-A1- 2 380 517**
**WO-A1-2012/082249      US-A1- 2008 071 267**
**US-A1- 2008 161 794**

**EP 2 604 209 B1**

**Beschreibung**

Die Erfindung betrifft eine Spülkathetereinrichtung, insbesondere einen Ablationskatheter, mit einem Katheterkörper, in dem sich ein zentrales Lumen von einem proximalen Bereich bis in einen distalen Bereich erstreckt, wobei vom Lumen eine Vielzahl von Spülkanälen abgeht, deren Austrittsöffnungen aus dem Katheterkörper über dessen distalen Endbereich verteilt sind. Sie betrifft des Weiteren eine Ablationsanordnung.

Ablationskatheter, mit denen auf elektrothermischen Wege und mit allen Vorteilen der minimal invasiven Chirurgie punkgenau Körpergewebe in Gefäßen oder Hohlorganen nekrotisiert bzw. abgetragen werden kann, sind seit langem bekannt und im klinischen Einsatz. Sie dienen u. a. zur Entfernung von ausgewählten Bereichen des reizbaren Herzgewebes im Rahmen der Therapie von Herzrhythmusstörungen. Für ihren erfolgreichen Einsatz ist es von erheblicher, vielfach von entscheidender Bedeutung, dass der Operateur die Güte des Kontaktes zwischen einer am distalen Katheterende angebrachten Elektrode oder einem anderen Ablationselement und dem Körpergewebe beurteilen kann.

Es sind auch Ablationskatheter bekannt, die über Spülkanäle mit Austrittsöffnungen (Spülöffnungen) im distalen Bereich versehen sind, welche dafür gedacht sind, Flüssigkeiten der Behandlungsstelle abzugeben, um umgebendes Gewebe an dieser Stelle zu kühlen oder ggfs. auch abgelöste Gewebsbestandteile abzuspülen. Die Spülkanäle sind über ein zentrales Lumen im Katheterkörper am proximalen Ende des Katheters an eine Pumpe angeschlossen, welche für die Flüssigkeitszufuhr aus einem Spülflüssigkeits-Reservoir sorgt. Ein derartiger Ablationskatheter, der nachfolgend auch als Spülkathetereinrichtung bezeichnet wird, wird beispielsweise in der EP 2 343 022 A1 beschrieben.

Der Erfindung liegt die Aufgabe zugrunde, eine verbesserte Spülkathetereinrichtung anzugeben, die zugleich als Mittel zur Bestimmung des Vorhandenseins und der Güte eines Kontaktes mit umliegendem Körpergewebe nutzbar ist.

Diese Aufgabe wird durch eine Spülkathetereinrichtung mit den Merkmalen des Anspruchs 1 gelöst. Zweckmäßige Fortbildungen des Erfindungsgedankens sind Gegenstand der abhängigen Ansprüche. Es wird des Weiteren eine Ablationsanordnung mit den Merkmalen des Anspruchs 12 bereitgestellt.

Die Erfindung schließt den Gedanken ein, zur Bestimmung der Güte des Gewebekontaktes vergleichende Druck- und/oder Durchflussmessungen in/an den Spülkanälen zu nutzen und im/am Spül- bzw. Ablationskatheter hierzu geeignete Mittel vorzusehen. Durch den Verschluss einzelner Spülkanäle durch anliegendes Gewebe erfolgt eine Veränderung der Druckverhältnisse. Interpretationsprinzip: Je mehr Spülkanäle durch Gewebe gedrosselt werden, desto größer ist die Auflagefläche/elektrisch wirksame Fläche der Elektrode. Da die Geometrie der Elektrode und der Spülkanäle bekannt ist kann der Gewebekontakt lokalisiert werden (z. B. Kontakt an der Spitze, seitlicher Kontakt). Hierdurch könnte ebenfalls eine Ermittlung des Auflagewinkels erfolgen.

Bei der vorgeschlagenen Spülkathetereinrichtung sind dem zentralen Lumen und/oder den einzelnen Spülkanälen Fühler zur Druckmessung oder zur Messung der Fließgeschwindigkeit zugeordnet. An die Fühler sind Auswertungsmittel angeschlossen, welche zur Bestimmung des Offen- oder Verschlossen-Zustandes eines Teils der Spülkanäle aufgrund von durch die Fühler erfassten Messwerten ausgebildet sind. Die Spülkathetereinrichtung gemäß des Gegenstandes der Patentanmeldung zeichnet sich dadurch aus, dass die sich im Katheterkopf befindenden Spülkanäle je nach Ausrichtung im Kopf unterschiedliche Durchmesser und/oder Längen auf weisen, damit das Schließen eines Spülkanals bei einem bestimmten Kontaktwinkel eine einzigartige Kombination von offenen Spülkanälen erzeugt, was wiederum einen einzigartigen Druckabfall bei konstanter Durchflussmenge im Katheter ermittelt.

Die Messgröße wird mittels eines oder mehrerer Drucksensoren erfasst. Es sind insbesondere drei Anbindungen der Messsensoren denkbar.

1.) Die Messung der Druckänderung erfolgt durch einen Drucksensor im Spülsystem, der die Veränderung im gesamten Spülsystem aufnimmt. Durch den Vergleich mit einer Referenzmessung wird auf die Kontaktgüte geschlossen

a. Variabler Druckabfall an einer Stelle gemessen.
b. Vorgegebene Durchflussrate.
c. Keine Mehrdeutigkeit durch variable Durchmesser in Düsen.

2.) Die Messung der Druckänderung erfolgt zeitgleich für mehrere Spülkanalbindungen. Dies ermöglicht eine sehr genaue Zustandsbestimmung.

a. Düse offen: Messungen statischen Drucks ist gleich.
b. Düsen geschlossen: Messungen statischen Drucks sind unterschiedlich. Statischer Druck in verschlossenen Kanälen ist höher.
c. Dynamischer Druck bleibt konstant im zuführenden Rohr, da die Durchflussrate vorgeben ist.
d. Digitale Messung

3.) Drehender Spülkopf, und mechanisches Verschlusselement (Multiplexer).

    a. Suchbetrieb; welche Düsen sind verstopft?
    b. Spülbetrieb; alle Düsen sind geöffnet.

Gemäß dem ersten der genannten Wirkprinzipien ist in einer Ausführung der Spülkathetereinrichtung nur dem zentralen Lumen ein Lumen-Fühler zugeordnet, und in den Auswertungsmitteln sind Vergleichermittel zum Vergleich aktuell erfasster Messwerte mit gespeicherten Vergleichswerten für die möglichen Kombinationen von Offen- und Verschlossen-Zuständen der Spülkanäle vorgesehen.

Gemäß einer Ausführung des zweiten Wirkprinzips ist jeweils einem Spülkanal oder einer Gruppe von Spülkanälen mit zueinander benachbarten Austrittsöffnungen ein Spülkanal-Fühler zugeordnet. In den Auswertungsmitteln sind hierbei Vergleichermittel zum Vergleich aktuell erfasster Messwerte mit gespeicherten Vergleichswerten von Offen- und Verschlossen-Zuständen des Spülkanals oder der Gruppe von Spülkanälen vorgesehen, der/die dem jeweiligen Spülkanal-Fühler zugeordnet ist/sind. In einer weiteren Ausgestaltung dieses Funktionsprinzips ist dem zentralen Lumen ein Lumen-Fühler und jeweils einen Spülkanal oder einer Gruppe von Spülkanälen mit zueinander benachbarten Austrittsöffnungen ein Spülkanal-Fühler zugeordnet, und in den Auswertungsmitteln sind Vergleichermittel zum Vergleich aktuell erfasster Messwerte der Spülkanal-Fühler mit aktuell erfassten Messwerten des Lumen-Fühlers vorgesehen.

Gemäß dem dritten o. g. Wirkprinzip ist im Lumen ein steuerbares mechanisches Verschlusselement zum selektiven Öffnen jeweils eines Spülkanal-Abgangs oder einer Gruppe von Spülkanal-Abgängen von Spülkanälen mit zueinander benachbarten Austrittsöffnungen angeordnet. Diesem Verschlusselement ist ein Verschlusselement-Fühler zugeordnet, und in den Auswertungsmitteln sind Vergleichermittel zum Vergleichen aktuell erfasster Messwerte mit gespeicherten Messwerten für einen Offen- und Verschlossen-Zustand jeweils in Zuordnung zu einem selektiv geöffneten Spülkanal-Abgang oder einer Gruppe geöffneter Spülkanal-Abgänge vorgesehen.

In einer Ausgestaltung dieser Ausführung ist das mechanische Verschlusselement als Hohlzylinder mit entsprechend der Position eines Spülkanal-Abgangs oder der Positionen einer Gruppe von Spülkanal-Abgängen angeordneten Wandungs-Öffnungen ausgebildet. Insbesondere ist das Verschlusselement aus hochgradig biegsamem Material gebildet. In einer weiteren Ausgestaltung ist zur Steuerung des Verschlusselementes ein an dessen proximales Ende angesetztes Kraftübertragungselement, insbesondere ein Draht oder Schlauch, vorgesehen.

Für alle genannten Varianten ist eine Messung des intrakardialen Drucks als Referenzdruck sinnvoll, wenn nicht zur Gewinnung verlässlicher Ergebnisse erforderlich. Hierzu ist ein vom distalen Endbereich bis zum proximalen Ende des Katheterkörpers durchgehender separater Vergleichsmesskanal vorgesehen, dem ein Vergleichs-Druckfühler zur Erfassung eines Gefäß- oder Hohlorgan- Innendruckwertes zugeordnet ist. Die Vergleichsdruckmessung erfolgt also über einen nach außen führenden Kanal im Katheter.

In einer weiteren Ausführung ist der oder jeder Spülkanal-Fühler zur Erfassung des dynamischen Drucks und/oder der Lumen-Fühler zur Erfassung des statischen Drucks ausgebildet. In einer noch anderen Ausführung der Erfindung ist der jeweilige Messwandler des Fühlers oder mindestens eines Teils der Fühler am proximalen Ende des Katheterkörpers außerhalb des Patienten angeordnet.

Die vorgeschlagene Ablationsanordnung umfasst neben einem gemäß zumindest einem Teil der obigen Aspekte ausgestalten Ablationskatheter ein Steuergerät mit einer Verarbeitungs- und Anzeigeeinrichtung, welche zur Verarbeitung der Auswertungsergebnisse zur Bestimmung der Güte des Gewebekontakts eines Ablationselements des Ablationskatheters und zur Bereitstellung einer entsprechenden Anzeige ausgebildet ist. In der Praxis erfolgt die Auswertung der Messwerte über eine Messkarte und wird an einem PC, Notebook oder Tablett-Computer mit entsprechender Softwareausstattung im Sinne klarer Handhabungs-Hinweise für den Operateur ("guter Kontakt-Ablation sinnvoll; schlechter Kontakt Kontakt-Ablation nicht sinnvoll") visualisiert.

Vorteile und Zweckmäßigkeiten der Erfindung ergeben sich aus der nachfolgenden Beschreibung von Ausführungsbeispielen der Erfindung anhand der Figuren. Von diesen zeigen:

Fig. 1        eine schematische Darstellung einer Ausführungsform der erfindungsgemäßen Ablationsanordnung in Art eines Blockschaltbildes,

Fig. 2        eine perspektivische Darstellung ("Hidden Line View") des distalen Endes einer Ausführungsform der Spülkathetereinrichtung,

Fig. 3A bis 3C    Ansichten zur Erläuterung der Funktionsweise der Spülkathetereinrichtung,

Fig. 4        eine schematische Längsschnittdarstellung des Katheterkopfes mit Angaben zum Messprinzip und

Fig. 5A bis 5E    schematische Längsschnittdarstellungen des Katheterkopfes eines weiteren erfindungsgemäßen Ablationskatheters, der nicht Gegenstand dieser Patentanmeldung ist.

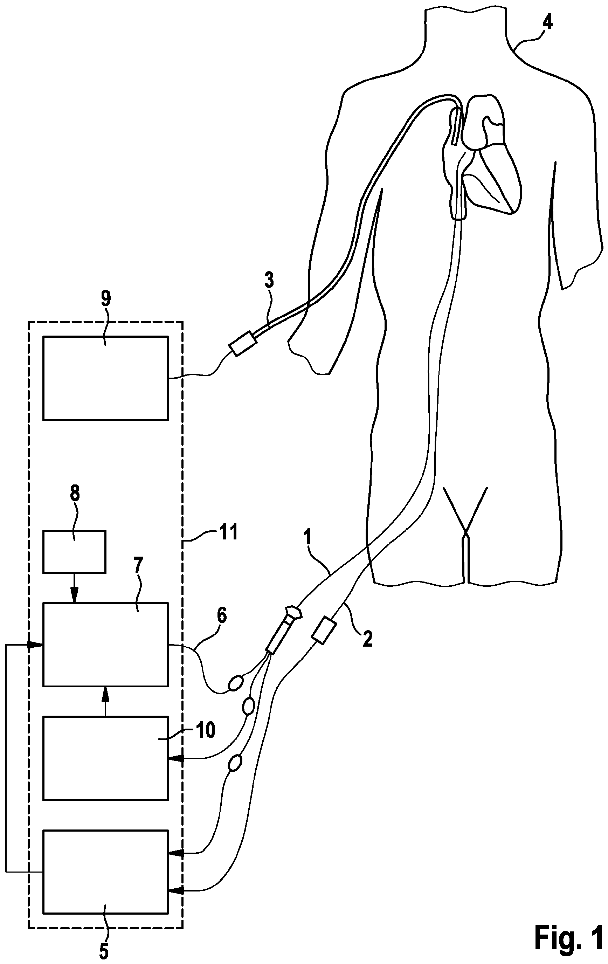

Fig. 1 zeigt in Art eines Blockschaltbildes eine Ablationsanordnung, die neben einem Ablationskatheter 1 mindestens einen weiteren EP-Diagnosekatheter 2 und einen Blutdruckmesskatheter 3 umfasst, deren distale Enden im Herzen eines Patienten 4 positioniert sind. Der Ablationskatheter 1 und der EP-Diagnose-Katheter 2 sind an ein EKG-Messsystem 5 angeschlossen. Der Ablationskatheter 1 verfügt (was weiter unten genauer erläutert wird) über Spülkanäle und -öffnungen, die dafür gedacht sind, Flüssigkeit an der Ablationsstelle abgeben zu können, um das Körpergewebe an dieser Stelle zu kühlen. Der Ablationskatheter 1 ist hierzu an einen Spülflüssigkeitsschlauch 6 angeschlossen, welcher über eine Pumpe 7 mit einen Flüssigkeitsreservoir 8 verbunden ist.

Das proximale Ende des Ablationskatheters 1 ist des Weiteren mit einer Verarbeitungs- und Anzeigeeinheit 10 verbunden, welche zur Verarbeitung und Anzeige der Ergebnisse von Druck- bzw. Durchflussmessungen ausgebildet ist, die im bzw. am Ablationskatheter ausgeführt werden, um die Güte eines Kontakts zwischen distalem Ende und anliegendem Körpergewebe zu bestimmen. Sowohl das EKG-Messsystem 5 als auch die Verarbeitungs- und Anzeigeeinheit 10 können über Steuersignalleitungen mit der Pumpe 7 verbunden sein und bilden zusammen mit dieser und dem Flüssigkeitsreservoir 8 sowie einem Blutdruckmessgerät 9, welches an den Blutdruckmesskatheter 3 angeschlossen ist, ein Therapiegerät 11 der Anordnung.

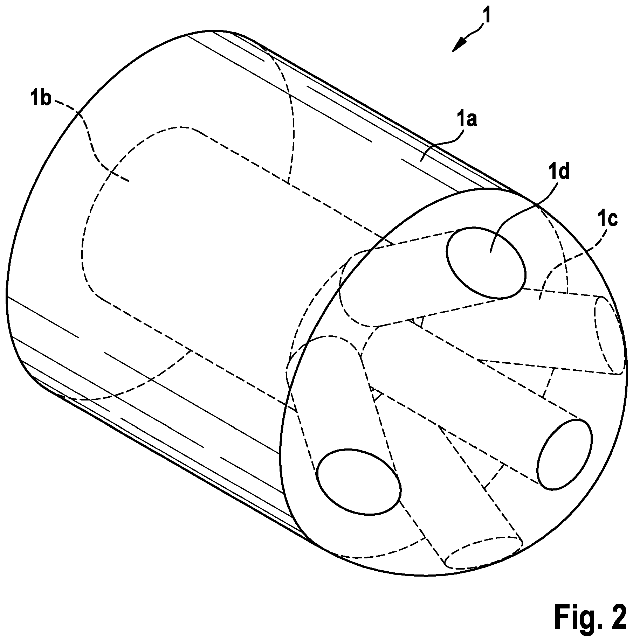

Fig. 2 zeigt das distale Ende des Ablationskatheters 1 bzw. dessen "Katheterkopf" 1a in einer Weise, dass nur die für die Erläuterung der Erfindung wesentliche Elemente zu erkennen sind, und zwar ein zentrales Lumen 1b und eine Mehrzahl von aus diesem abgehenden Spülkanälen 1c, die auf der äußeren Oberfläche des Katheterkopfes 1a jeweils eine Austrittsöffnung 1d haben.

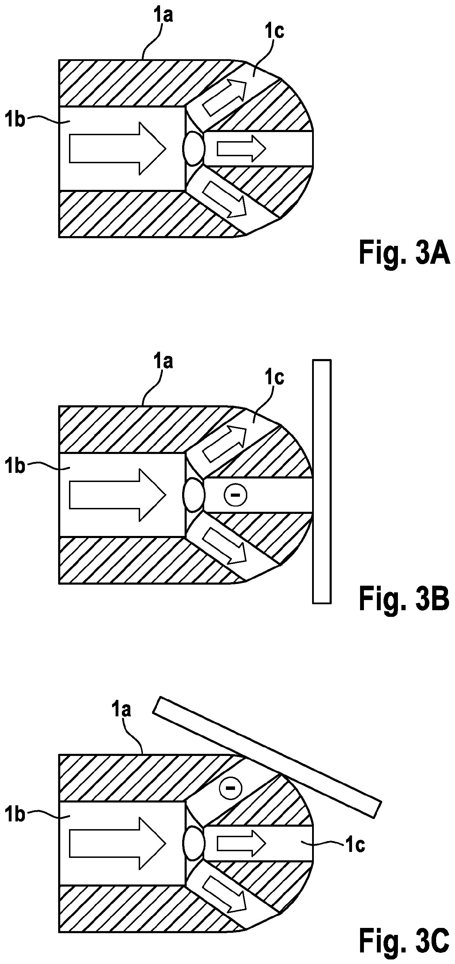

Diese Bezeichnungsweise ist auch in den Figuren 3A bis 3C gewählt, in denen schematisch dargestellt ist, wie ein Kontakt des Katheterkopfes 1a mit anliegendem Körpergewebe in einer bestimmten Winkellage zu einem Verschluss eines Teils der Austrittsöffnungen der Spülkanäle 1c führt. Es leuchtet unmittelbar ein, dass ein derartiger Verschluss zu einer Erhöhung des im jeweiligen Spülkanal messbaren Drucks bzw. zu einer Verringerung der messbaren Fließgeschwindigkeit (bis auf Null) führt.

Die Ermittlung des Kontaktwinkels erfolgt durch die Differenzierung des gemessenen Druckabfalls bei vorgegebener Durchflussmenge. Wenn die Durchflussmenge konstant bleibt, hängt der Druckabfall nur von der Kombination der offenen Spülkanäle im Katheterkopf ab. Der niedrigste Druckabfall bei konstanter Durchflussmenge wird gemessen, wenn alle Spülkanäle offen sind. Das Schließen eines Spülkanals bei konstanter Durchflussmenge im Katheter erhöht die Durchflussmenge in den offenen bleibenden Spülkanälen und demzufolge wird der im Katheterschlauch (oder an der Pumpe) gemessene Druckabfall höher. Die Erhöhung des Druckabfalls (gegen Druck im Herzen gemessen) im Katheterschlauch deutet im Allgemeinen auf Kontakt des Katheterkopfes am Gewebe hin.

Die sich im Katheterkopf befindenden Spülkanäle weisen je nach Ausrichtung im Kopf unterschiedliche Durchmesser und/oder Längen auf, damit das Schließen eines Spülkanals bei einem bestimmten Kontaktwinkel eine einzigartige Kombination von offenen Spülkanälen erzeugt, was wiederum einen einzigartigen Druckabfall bei konstanter Durchflussmenge im Katheter ermittelt.

Der Druckabfall im Katheterschlauch lässt sich mit der folgenden Formel berechnen:

$$\Delta P = \frac{128\mu LQ}{\pi d^4}$$

worin bedeuten:

$\Delta P$: Druckabfall.
$\mu$: Dynamische Viskosität, Wasser = 0.8e-3 Pa-s.
$L$: Länge des Kanals.
$Q$: Durchflussmenge, Wasser = 20 ml/min.
$d$: Durchmesser des Kanals.

In der obigen Formel ist die Abhängigkeit des Druckabfalls vom Durchmesser und von der Länge eines Kanals ersichtlich. In dieser Berechnung des Druckabfalls wurden die sogenannten "Minor Losses" vom Druckabfall nicht berücksichtigt. "Minor Losses" beinhalten die Druckabfälle durch Verengungen, Umlenkungen, Ausweitungen und Austrittsöffnungen im Kanal.

Der Druckabfall in den sich im Katheterkopf befindenden Spülkanälen wird aber anders berechnet. Da die Mehrheit des Druckabfalls durch Umlenkungen,
Verengungen usw. erzeugt wird, benutzt man die Formel für "Minor Losses".

$$\Delta P = 0.5\xi v^2$$

worin bedeuten:

$\xi$:  Druckabfallkoeffizient.

v:  Durchschnittliche Flussgeschwindigkeit.

Tabelle 1 stellt beispielhafte Abmessungen eines fiktiven Katheters dar, der drei verschiedene Flussabschnitte hat, mit beispielhaften Druckabfallkoeffizienten.

**Tabelle 1. Abmessungen der Flussabschnitte im Katheter bzw. Katheterkopf.**

|  | Katheterschlauch | Spülkanal Mitte | Spülkanal Seitlich |
|---|---|---|---|
| Anzahl | 1 | 1 | 4 |
| Durchmesser (mm) | 1.00 | 0.40 | 0.30 |
| Länge (mm) | 1500 | - | - |
| Druckabfall-koeffizient (-) | - | 1.0e4 | 1.0e4 |

Die Druckabfälle der einzelnen Flussabschnitte wurden für drei Szenarien berechnet und in Tabelle 2 aufgelistet. Bei allen Szenarien handelt es sich um eine Durchflussmenge von 20 ml/min. Die drei Szenarien sind:

1. Alle Spülkanäle offen.
2. Spülkanäle in der Mitte durch Kontakt mit Gewebe um 90° geschlossen.
3. Ein seitlich angebrachter Spülkanal durch Kontakt mit Gewebe um etwa 45° geschlossen.

**Tabelle 2. Berechnung der Druckabfälle in drei Szenarien.**

| Druckabfall (mbar) | Szenario 1 | Szenario 2 | Szenario 3 |
|---|---|---|---|
| Katheterschlauch | 163.0 | 163.0 | 163.0 |
| Spülkanal Mitte | 33.3 | -zu- | 48.7 |
| Spülkanal Seitlich | 33.3 | 69.5 | 1 Kanal zu |
| **Total** | **196.3** | **232.5** | **211.7** |

Aus Tabelle 2 wird das Grundprinzip ersichtlich: 1. Der Druckabfall im Katheterschlauch bleibt konstant, denn die Durchflussmenge ist von der Pumpe vorgegeben. Wenn alle Kanäle offen sind, mißt der Druckabfall an der Pumpe 196.3 mbar.
2. Das Sperren des mittleren Kanals (Szenario 2) erhöht den an der Pumpe gemessenen Druckabfall auf 232.5 mbar. Dieses Szenario entspricht einem Kontakt mit dem Gewebe um 90°.
3. Das Sperren eines der 4 seitlich angebrachten Spülkanals erhöht ebenfalls den an der Pumpe gemessenen Druckabfall, aber weniger, nur auf 211.7 mbar. Dieses Szenario entspricht einem seitlichen Kontakt am Gewebe um ca. 45°.

Da sich der Druckabfall in diesen Szenarien klar unterscheidet, wird es nur anhand des ermittelten Druckabfalls eindeutig ersichtlich, ob es sich um einen Kontakt mit dem Gewebe um 90° oder 45° handelt.
Die Druckabfallkoeffizienten der Spülkanäle können fein abgestimmt sein, um den maximalen Druckunterschied zu erzeugen, damit das ermittelte Signal deutlich wird. Das Abstimmen kann durch Einbauen von Verengungen order Umlenkungen erfolgen.
Zur Bestimmung des Gewebekontakts zwischen der Katheterspitze und dem umgebenden Gewebe wird in einer weiteren Ausgestaltung während des Spülvorgangs die Strömungsgeschwindigkeit in den Spülkanälen überwacht: Bei gutem Gewebekontakt sind die Spülkanäle in der Kontaktzone verschlossen und die Strömungsgeschwindigkeit in den entsprechenden Kanälen ist deutlich kleiner als in den nicht verschlossenen Kanälen. Somit kann aus den Strömungsgeschwindigkeiten in den Spülkanälen die Lage und Größe des Gewebekontakts bestimmt werden.

Zur Druckmessung stehen prinzipiell viele Methoden zur Verfügung, wobei nicht alle sinnvoll miniaturisiert werden können. Besonders geeignet sind Verfahren, bei denen die eigentliche Geschwindigkeitsmessung außerhalb des Katheters durchgeführt werden kann.

Dies ist z.B. mit einem Druckmessverfahren möglich: Nach der Bernoulli Gleichung kann die Strömungsgeschwindigkeit aus der Druckdifferenz zwischen statischem und dynamischen Druck bestimm werden. Dieser Effekt wird beim Pitot-Rohr genutzt.

Im Katheter bietet sich eine andere Vorgehensweise an, um die dünnen Spülkanäle nicht unnötig zu verengen. Der statische Druck kann statt mit einem Pitot-Rohr indirekt über die bekannte Flussrate und die Messung des dynamischen Drucks kurz vor der Verzweigung gemessen werden. Kurz nach der Verzweigung kann in den Spülkanälen erneut der dynamische Druck gemessen werden. Wenn beide Messpunkte nahe beieinander liegen, ist der Druckabfall vernachlässigbar klein.

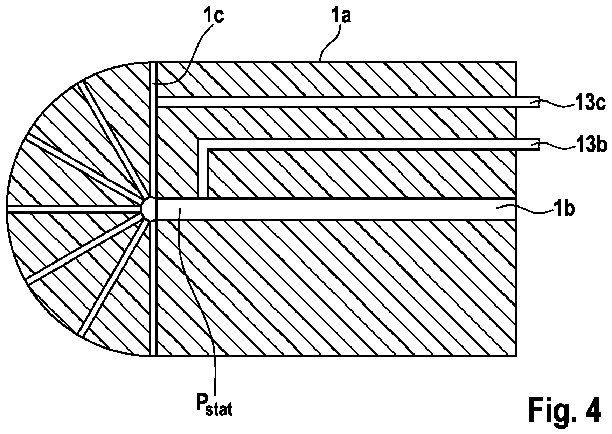

In Fig. 4 ist nochmals symbolisch der Katheterkopf 1a gezeigt, der ein zentrales Lumen 1b zur Spülkanalversorgung besitzt, welches sich in verschiedene Spülkanäle 1c verzweigt. Die Messung des statischen Druckes erfolgt über eine dynamische Druckmessung mit der Flüssigkeitssäule 13b unter Berücksichtung der bekannten Flussrate (vom proximalen Katheterende). Die Druckbestimmung in den einzelnen Spülkanälen erfolgt mit mehreren Flüssigkeitssäulen 13c, wobei exemplarisch nur eine angebildet ist. Aus den gemessenen dynamischen Drücken in den Spülkanälen und dem statischen Druck vor der Verzweigung können die Strömungsgeschwindigkeiten in jedem Kanal bestimmt werden. Alle Druckwandler für die Flüssigkeitssäule 13b und die Flüssigkeitssäulen 13c befinden sich außerhalb des Patienten.

Ausgehend von dem oben beschriebenen Konzept können die Flüssigkeitssäulen mehrerer benachbarter Spülkanäle kombiniert werden, um die Anzahl der Flüssigkeitssäulen im Katheter zu verringern. So erfolgt die Messung nicht mehr pro Kanal sondern pro Areal. 5 Areale können z.B. die Spitze und 4 radikal angeordnete proximale Areale sein.

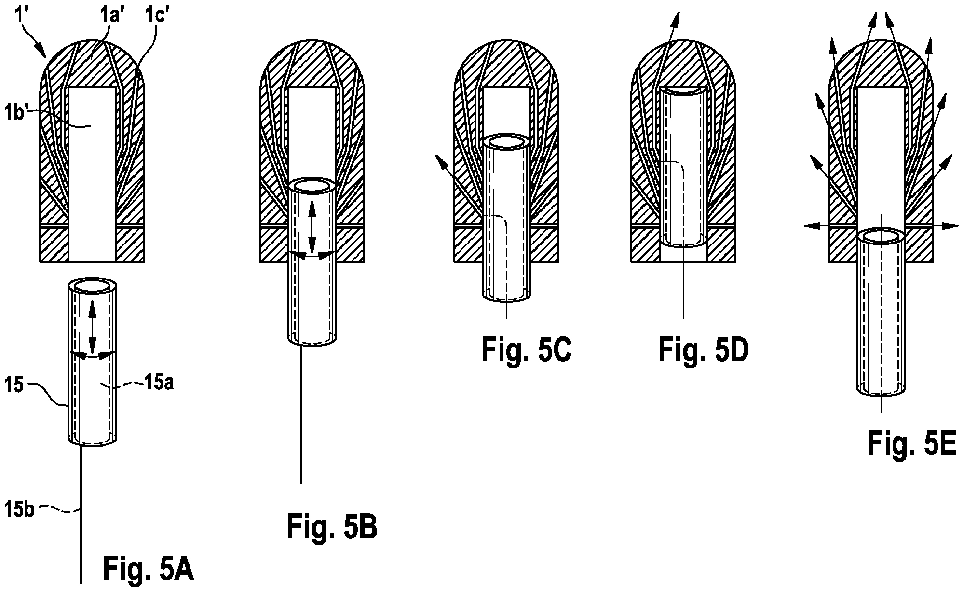

Fig. 5A bis 5E zeigen als weiteres Ausführungsbeispiel einer Erfindung, die nicht Gegenstand dieser Anmeldung ist, den Aufbau und verschiedene Gebrauchszustände des Katheterkopfes eines weiteren Ablationskatheters 1'. Im Interesse besserer Übersichtlichkeit sind nur in Fig. 5A Bezugsziffern angegeben. Die Bezugsziffern sind in Anlehnung an Fig. 2 bis 3C gewählt.

Im Katheterkopf 1a' ist ein mechanisches Verschlusselement 15 mit der Grundform eines Hohlzylinders vorgesehen, welches in das Lumen 1b' passt und eine Öffnung 15a aufweist, welche mittels eines am proximalen Ende des Hohlzylinders 15 angebrachten (vom proximalen Katheterende aus handhabbaren) Positionierungsdrahtes 15b in Überdeckung mit dem Abgang eines gewünschten Spülkanals 1c' aus dem Lumen 1b' gebracht werden kann.

Beispiele für die Auswahl einzelner Spülkanäle mittels des Verschlusselementes 15 sind in Fig. 5B bis 5D gezeigt, wobei in Fig. 5C und 5D jeweils auch ein Flüssigkeitsstrom aus dem Lumen über einen ausgewählten Spülkanal und dessen Austrittsöffnung bis in den Bereich distal von der Katheterspitze gezeigt ist. In Fig. 5E ist eine Position des Verschlusselementes gezeigt, in der alle Spülkanäle mit Spülflüssigkeit beaufschlagt werden, so dass aus allen Austrittsöffnungen Spülflüssigkeit austritt.

Die Verschlusseinrichtung wird durch Dreh- und Vorschubbewegungen in einer der Anzahl der Spülkanäle entsprechenden Anzahl von Schritten jeweils so positioniert, dass je ein Spülkanal auf Durchgang ("aktiv") geschaltet ist, während alle anderen Spülkanäle verschlossen bleiben. In jeder der Suchpositionen wird eine Druck- bzw. Durchflussmessung ausgeführt, um festzustellen, ob der ausgewählte Spülkanal offen oder verschlossen ist. Um anschließend den üblichen Spülbetrieb über alle Spülkanäle zu gewährleisten, wird das Verschlusselement in den in Fig. 5E gezeigten Zustand gebracht.

Die Ausführung der Erfindung ist nicht auf die oben beschriebenen Beispiele und hervorgehobenen Aspekte beschränkt, sondern ebenso in einer Vielzahl von Abwandlungen möglich, die im Rahmen fachgemäßen Handelns liegen.

## Patentansprüche

1. Spülkathetereinrichtung, insbesondere Ablationskatheter (1), mit

- einem Katheterkörper, in dem sich ein zentrales Lumen (1b) von einem proximalen Bereich bis in einen distalen Bereich erstreckt, wobei vom Lumen eine Vielzahl von Spülkanälen (1c) abgeht, deren Austrittsöffnungen (1d) aus dem Katheterkörper über dessen distalen Endbereich (1a) verteilt sind, und
- Auswertungsmittel,

wobei dem zentralen Lumen und/oder den einzelnen Spülkanälen mindestens ein Fühler zur Druckmessung oder zur Messung der Fließgeschwindigkeit zugeordnet sind, an den die Auswertungsmittel angeschlossen sind, welche zur Bestimmung des Offen- oder Verschlossen-Zustandes eines Teils der Spülkanäle aufgrund von durch den Fühler erfassten Messwerten ausgebildet sind,
**dadurch gekennzeichnet, dass**

die sich im distalen Endbereich befindenden Spülkanäle je nach Ausrichtung im distalen Endbereich unterschiedliche Durchmesser und/oder Längen aufweisen.

2. Spülkathetereinrichtung nach Anspruch 1, **dadurch gekennzeichnet, dass** nur dem zentralen Lumen (1b) ein Lumen-Fühler zugeordnet ist und in den Auswertungsmitteln Vergleichermittel zum Vergleich aktuell erfasster Messwerte mit gespeicherten Vergleichswerten für die möglichen Kombinationen von Offen- und Verschlossen-Zuständen der Spülkanäle vorgesehen sind.

3. Spülkathetereinrichtung nach Anspruch 1, **dadurch gekennzeichnet, dass** jeweils einem Spülkanal (1c) oder einer Gruppe von Spülkanälen (1c) mit zueinander benachbarten Austrittsöffnungen (1d) ein Spülkanal-Fühler zugeordnet ist und in den Auswertungsmitteln Vergleichermittel zum Vergleich aktuell erfasster Messwerte mit gespeicherten Vergleichswerten von Offen- und Verschlossen-Zuständen des Spülkanals oder der Gruppe von Spülkanälen vorgesehen sind, der/die dem jeweiligen Spülkanal-Fühler zugeordnet ist/sind.

4. Spülkathetereinrichtung nach Anspruch 1, **dadurch gekennzeichnet, dass** dem zentralen Lumen (1b) ein Lumen-Fühler und jeweils einen Spülkanal (1c) oder einer Gruppe von Spülkanälen (1c) mit zueinander benachbarten Austrittsöffnungen (1d) ein Spülkanal-Fühler zugeordnet ist und in den Auswertungsmitteln Vergleichermittel zum Vergleich aktuell erfasster Messwerte der Spülkanal-Fühler mit aktuell erfassten Messwerten des Lumen-Fühlers vorgesehen sind.

5. Spülkathetereinrichtung nach einem der vorangehenden Ansprüche, **dadurch gekennzeichnet, dass** die Spülkanäle Verengungen oder Umlenkungen aufweisen.

6. Spülkathetereinrichtung nach einem der vorangehenden Ansprüche, **dadurch gekennzeichnet, dass** ein vom distalen Endbereich (1a) bis zum proximalen Ende des Katheterkörpers durchgehender separater Vergleichsmesskanal vorgesehen ist, dem ein Vergleichs-Druckfühler zur Erfassung eines Gefäß- oder Hohlorgan- Innendruckwertes zugeordnet ist.

7. Spülkathetereinrichtung nach einem der vorangehenden Ansprüche, **dadurch gekennzeichnet, dass** der oder jeder Spülkanal-Fühler zur Erfassung des dynamischen Drucks und/oder der Lumen-Fühler zur Erfassung des statischen Drucks ausgebildet ist.

8. Spülkathetereinrichtung nach einem der vorangehenden Ansprüche, **dadurch gekennzeichnet, dass** der jeweilige Messwandler des Fühlers oder mindestens eines Teils der Fühler am proximalen Ende des Katheterkörpers außerhalb des Patienten angeordnet ist.

9. Ablationsanordnung, mit einer als Ablationskatheter ausgebildeten Spülkathetereinrichtung nach einem der vorangehenden Ansprüche und einem Steuergerät (11), **dadurch gekennzeichnet, dass** die Auswertungsmittel der Spülkathetereinrichtung mit einer Verarbeitungs- und Anzeigeinrichtung (10) des Steuergerätes verbunden sind, welche zur Verarbeitung der Auswertungsergebnisse zur Bestimmung der Güte des Gewebekontakts eines Ablationselements des Ablationskatheters und zur Bereitstellung einer entsprechenden Anzeige ausgebildet ist.

**Claims**

1. An irrigation catheter device, in particular an ablation catheter (1), with

    - a catheter body in which a central lumen (1b) extends from a proximal area to a distal area, the lumen having multiple irrigation canals (1c) branching off from it, whose exit openings (1d) from the catheter body are distributed over its distal end area (1a), and
    - means of evaluation,

    the central lumen and/or the individual irrigation canals being associated with at least one sensor to measure the pressure or to measure the flow rate, to which are connected means of evaluation, which are designed to determine the open-or-closed state of a part of the irrigation canals on the basis of measurements detected by the sensor, **characterized in that** the irrigation canals located in the distal end area have different diameters and/or lengths in the distal end area, depending on the orientation.

**2.** An irrigation catheter device according to claim 1, **characterized in that** only the central lumen (1b) is associated with a lumen sensor and the means of evaluation have means of comparison to compare currently detected measurements with stored comparison values for the possible combinations of open-or-closed states of the irrigation canals.

**3.** An irrigation catheter device according to claim 1, **characterized in that** each irrigation canal (1c) or group of irrigation canals (1c) with adjacent exit openings (1d) is associated with an irrigation canal sensor, and the means of evaluation have means of comparison to compare currently detected measurements with stored comparison values of open-or-closed states of the irrigation canal or the group of irrigation canals, this/these stored comparison value(s) being associated with the respective irrigation canal sensor.

**4.** An irrigation catheter device according to claim 1, **characterized in that** the central lumen (1b) is associated with a lumen sensor, and each irrigation canal (1c) or group of irrigation canals (1c) with adjacent exit openings (1d) is associated with an irrigation canal sensor, and the means of evaluation having means of comparison to compare currently detected measurements of the irrigation canal sensor with currently detected measurements of the lumen sensor.

**5.** An irrigation catheter device according to one of the preceding claims, **characterized in that** the irrigation canals have narrowings or deflections.

**6.** An irrigation catheter device according any one of the preceding claims, **characterized in that** a separate comparison measurement canal is provided that runs continuously from the distal end area (1a) to the proximal end of the catheter body, which is associated with a pressure comparison sensor to detect a pressure inside a vessel or hollow organ.

**7.** An irrigation catheter device according to any one of the preceding claims, **characterized in that** the irrigation canal sensor, or every irrigation canal sensor, is designed to detect the dynamic pressure and/or the lumen sensor is designed to detect the static pressure.

**8.** An irrigation catheter device according to any one of the preceding claims, **characterized in that** the respective measuring transducer of the sensor or at least one part of the sensor is arranged at the proximal end of the catheter body outside the patient.

**9.** An ablation arrangement with an irrigation catheter device in the form of an ablation catheter according to any one of the preceding claims and a controller (11), **characterized in that** the means of evaluation of the irrigation catheter device are connected with a processing and display device (10) of the controller, which are designed to process the evaluation results to determine the quality of the tissue contact of an ablation element of the ablation catheter and to provide a corresponding display of it.

**Revendications**

**1.** Système de cathéter d'irrigation, notamment cathéter d'ablation (1) avec

- un corps de cathéter dans lequel une lumière (1b) centrale s'étend à partir d'une région proximale jusqu'à une région distale, où une multiplicité de canaux d'irrigation (1c) part de la lumière, dont les orifices de sortie (1d) sont répartis à partir du corps de cathéter sur sa région d'extrémité (1a) distale, et
- un moyen d'exploitation,

où, au niveau de la lumière centrale et/ou aux canaux d'irrigation individuels, moins une sonde est associée pour la mesure de la pression ou pour la mesure de la vitesse d'écoulement, à laquelle sont raccordés des moyens d'exploitation, lesquels sont conçus pour la détermination de l'état d'ouverture ou de fermeture d'une partie des canaux d'irrigation en raison de valeurs de mesure saisies par la sonde, **caractérisé en ce que** les canaux d'irrigation se trouvant dans la région de l'extrémité distale présentent des diamètres et/ou des longueurs, variables dans la région d'extrémité distale en fonction de l'orientation.

**2.** Système de cathéter d'irrigation selon la revendication 1, **caractérisé en ce qu'**une sonde de lumière est associée uniquement à la lumière (1b) centrale et des moyens de comparaison sont prévus dans les moyens d'exploitation pour la comparaison de valeurs de mesure instantanées saisies avec des valeurs de comparaison pour les combinaisons possibles d'états d'ouverture et de fermeture des canaux d'irrigation.

3. Système de cathéter d'irrigation selon la revendication 1, **caractérisé en ce que** respectivement un canal d'irrigation (1c) ou un groupe de canaux d'irrigation (1c) avec des orifices de sortie (1d) voisins les uns pour les autres sont associés avec une sonde de canal d'irrigation et des moyens de comparaison sont prévus dans les moyens d'exploitation pour la comparaison de valeurs de mesure instantanées saisies avec des valeurs de comparaison stockées d'états d'ouverture et de fermeture du canal d'irrigation ou du groupe de canaux d'irrigation, qui est/sont associé/s à la sonde de canal d'irrigation correspondante.

4. Système de cathéter d'irrigation selon la revendication 1, **caractérisé en ce qu'**une sonde de lumière est associée à la lumière (1b) centrale et une sonde de canal d'irrigation est respectivement associée à un canal d'irrigation (1c) ou à un groupe de canaux d'irrigation (1c) avec des orifices de sorties (1d) voisins les uns par rapport aux autres et des moyens de comparaison sont prévus dans les moyens d'exploitation pour la comparaison de valeurs de mesure instantanées saisies des sondes de canal d'irrigation avec des valeurs de mesure instantanées saisies de la sonde de lumière.

5. Système de cathéter d'irrigation selon l'une des revendications précédentes, **caractérisé en ce que** les canaux d'irrigation présentent des rétrécissements et des déviations.

6. Système de cathéter d'irrigation selon l'une des revendications précédentes, **caractérisé en ce qu'**un canal de mesure de comparaison séparé traversant est prévu à partir de la région d'extrémité (1a) distale jusqu'à l'extrémité proximale du corps de cathéter, canal de mesure auquel est associée une sonde de pression comparative pour la saisie d'une valeur de pression interne d'un vaisseau, ou d'un organe creux.

7. Système de cathéter d'irrigation selon l'une des revendications précédentes, **caractérisé en ce que** la, ou chaque, sonde de canal d'irrigation est conçue pour la saisie de la pression dynamique, et/ou la sonde de lumière est conçue pour la saisie de la pression statique.

8. Système de cathéter d'irrigation selon l'une des revendications précédentes, **caractérisé en ce que** l'autotransformateur de mesure de la sonde respectif ou au moins une partie de la sonde est disposé à l'extrémité proximale du corps de cathéter en dehors du patient.

9. Système d'ablation, avec un système de cathéter d'irrigation conçu sous la forme de cathéter d'ablation selon l'une des revendications précédentes, et un appareil de commande (11), **caractérisé en ce que** les moyens d'exploitation du système de cathéter d'irrigation sont relié avec une unité de traitement et d'affichage (10) de l'appareil de commande, lesquels sont conçus pour le traitement des résultats d'exploitation pour la détermination de la bonne qualité du contact avec le tissu d'un élément d'ablation du cathéter d'ablation et pour la fourniture d'un affichage correspondant.

**Fig. 1**

1

1b

1a

1d

1c

Fig. 2

Fig. 3A

Fig. 3B

Fig. 3C

1c    1a

13c

13b

1b

P$_{stat}$

Fig. 4

Fig. 5A

Fig. 5B

Fig. 5C

Fig. 5D

Fig. 5E

EP 2 604 209 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2343022 A1